(19) Europäisches Patentamt
European Patent Office
Office européen des brevets

(11) Veröffentlichungsnummer: **0 203 446 A1**

(12) **EUROPÄISCHE PATENTANMELDUNG**

(21) Anmeldenummer: **86106488.9**

(22) Anmeldetag: **13.05.86**

(51) Int. Cl.⁴: **C 07 C 93/14**, C 09 B 51/00, A 61 K 7/13

(30) Priorität: **30.05.85 DE 3519304**

(43) Veröffentlichungstag der Anmeldung: **03.12.86 Patentblatt 86/49**

(84) Benannte Vertragsstaaten: **AT BE CH DE FR GB IT LI LU NL SE**

(71) Anmelder: **Wella Aktiengesellschaft, Berliner Allee 65, D-6100 Darmstadt (DE)**

(72) Erfinder: **Braun, Hans-Jürgen, Dr., Impasse de la Colline 3, CH-1723 Marly (CH)**
Erfinder: **Konrad, Eugen, Mecklenburger Strasse 101, D-6100 Darmstadt (DE)**
Erfinder: **Mager, Herbert, Dr., Beaumont 5, CH-1700 Fribourg (CH)**

(54) **Nitro-p-phenylendiaminderivative, Verfahren zu ihrer Herstellung sowie Haarfärbemittel mit einem Gehalt an diesen Verbindungen.**

(57) Nitro-p-phenylendiaminderivate der allgemeinen Formel I

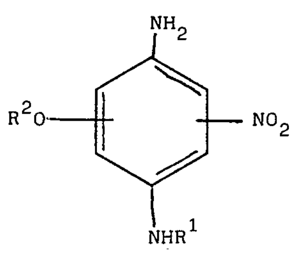

(I)

worin die Gruppe $R^2O$ sich in p-Stellung zur Nitrogruppe befindet und der Rest $R^1$ einen Alkyl-, Mono- oder Polyhydroxyalkyl-, Aminoalkyl- oder Mono- oder Dialkylaminoalkylrest bedeutet und der Rest $R^2$ unabhängig von $R^1$ einen Alkyl- oder Mono- oder Dihydroxyalkylrest darstellt und ein Verfahren zu deren Herstellung. Die Anmeldung betrifft ferner Mittel zur Färbung von Haaren mit einem Gehalt an Nitro-p-phenylendiaminderivaten der allgemeinen Formel I. Die Verbindungen der Formel I stellen direkt auf das Haar aufziehende, mit einem sehr reinen und leuchtenden Rotton färbende Haarfarbstoffe dar. Sie zeigen eine gute Farbechtheit und können sowohl in Haarfärbemitteln ohne Oxidationsmittelzugabe als auch in Oxidationshaarfärbemitteln eingesetzt werden.

EP 0 203 446 A1

## Beschreibung

### Nitro-p-phenylendiaminderivate, Verfahren zu ihrer Herstellung sowie Haarfärbemittel mit einem Gehalt an diesen Verbindungen

Gegenstand der Erfindung sind Mittel zur Färbung von Haaren mit Nitrofarbstoffen, wobei als Nitrofarbstoffe neue Derivate des Nitro-p-phenylendiamins verwendet werden sowie ein Verfahren zur Herstellung dieser Derivate.

Nitrofarbstoffe haben für die Haarfärbung eine wesentliche Bedeutung erlangt. Durch Kombination verschiedener Nitrofarbstoffe lassen sich Färbemittel herstellen, die ohne Zusatz von Oxidationsmitteln Haare in natürlichen oder in modischen Tönen zu färben vermögen. Die Nitrofarbstoffe sind auch wichtige Bestandteile von Oxidationshaarfärbemitteln, in denen sie zur Abrundung des Farbergebnisses und zur Erzeugung von modischen Farbnuancen herangezogen werden.

Zur Erzeugung von modischen Rotnuancen werden insbesondere Nitrofarbstoffe benötigt, die dem Haar eine intensive Rotfärbung verleihen können.

An Nitrofarbstoffe, die zum Färben von menschlichen Haaren Verwendung finden, sind zahlreiche besondere Anforderungen gestellt. So müssen sie in toxikologischer und dermatologischer Hinsicht unbedenklich sein und die Erzielung von Färbungen in der gewünschten Intensität ermöglichen, was unter anderem auch eine ausreichende Wasserlöslichkeit voraussetzt. Außerdem wird für die erzielten Haarfärbungen eine

gute Licht-, Säure- und Reibechtheit gefordert. Ihre Verwendung in Oxidationsfärbemitteln setzt weiterhin voraus, daß die Nitrofarbstoffe gegenüber Wasserstoffperoxid in ammoniakalischer Lösung und gegenüber Antioxidantien stabil sind.

Es sind schon mehrfach Derivate des Nitro-p-phenylendiamins beschrieben worden, die eine Haarfärbung im Rotbereich ermöglichen. Diese Verbindungen werden den oben genannten Anforderungen jedoch nur zum Teil gerecht.

Das Nitro-p-phenylendiamin selbst färbt das Haar in einem Rotton und wird daher in großem Umfang in Haarfärbemitteln eingesetzt. Die Leuchtkraft und Farbintensität des Nitro-p-phenylendiamins ist jedoch nicht zufriedenstellend.

In der DE-OS 21 57 844 werden Derivate des Nitro-p-phenylendiamins beschrieben, die das Haar in einem blaustichigen Rot einfärben. So erhält man zum Beispiel mit dem 5-Chlor-4-[(2'-hydroxyethyl)amino]-2-nitro-anilin eine weinrote Haarfärbung, die eine geringe Intensität aufweist. Ebenfalls eine blaurote Färbung erhält man mit der in der DE-OS 19 24 249 beschriebenen Verbindung $N^1$,$N^4$-Dimethyl-2-nitro-5-methoxy-p-phenylendiamin. Dagegen färbt das in der CA-PS 935 094 beschriebene 5-Methoxy-2-nitro-p-phenylendiamin das Haar in einen orangen Farbton.

Die oben beschriebenen Derivate des Nitro-p-phenylendiamins sind also auf Grund ihrer Färbeeigenschaften nicht geeignet, dieses in Haarfärbemitteln zu ersetzen.

Hierzu wurde nun gefunden, daß durch neue Nitro-p-phenylendiaminderivate der allgemeinen Formel I

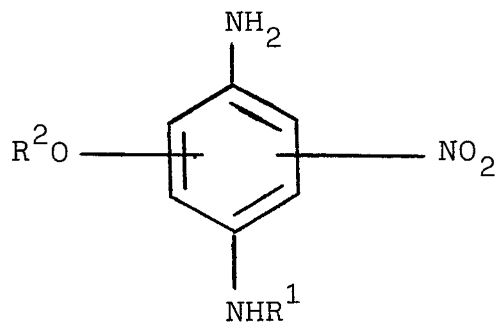

(I),

worin die Gruppe $R^2O$ sich in p-Stellung zur Nitrogruppe befindet und der Rest $R^1$

- einen Alkylrest mit 1 bis 6 Kohlenstoffatomen oder

- einen Mono- oder Polyhydroxyalkylrest mit 1 bis 6 Kohlenstoffatomen und bis zu 3 Hydroxylgruppen oder

- einen Aminoalkylrest oder einen Mono- oder Dialkylaminoalkylrest der allgemeinen Formel $C_mH_{2m}N(C_nH_{2n})_pH_{(2-p)}$, wobei m und n unabhängig voneinander eine ganze Zahl von 1 bis 6 darstellen und p eine ganze Zahl von 0 bis 2 ist,

bedeutet und der Rest $R^2$ unabhängig vom Rest $R^1$

- einen Alkylrest mit 1 bis 6 Kohlenstoffatomen oder

- einen Mono- oder Dihydroxyalkylrest mit 1 bis 6 Kohlenstoffatomen

darstellt, die gestellte Aufgabe in hervorragender Weise gelöst wird.

Beispiele für erfindungsgemäße Verbindungen der Formel I sind

2-Nitro-4-[(2'-hydroxyethyl)amino]-5-methoxy-anilin,

2-Nitro-4-[(2'-hydroxyethyl)amino]-5-(2''-hydroxyethoxy)-anilin,

5-Nitro-4-[(2'-hydroxyethyl)amino]-2-methoxy-anilin,

2-Nitro-4-[(2',3'-dihydroxypropyl)amino]-5-methoxy-anilin und

2-Nitro-4-[(2'-hydroxyethyl)amino]-5-(2'',3''-dihydroxypropoxy)-anilin.

Die Verbindungen der Formel I vermögen das Haar in einem leuchtenden Rotton in überraschender Farbreinheit zu färben. Die Färbungen sind weder blaustichignoch orangestichig-rot. Daneben werden noch weitere Vorteile gegenüber den bekannten Farbstoffen beobachtet.

Die Intensität der Färbungen, die man mit den neuen Verbindungen erhält, ist deutlich höher als die Intensität der Färbungen mit den bekannten roten Nitrofarbstoffen. So ist beispielsweise die Intensität der Färbung, die man mit 2-Nitro-4-[(2'-hydroxyethyl)amino]-5-methoxy-anilin erhält, gegenüber der Intensität der Färbung mit Nitro-p-phenylendiamin, um den Faktor 2 gesteigert.

Dieser Befund ist umso überraschender, als man normalerweise bei der Einführung eines weiteren Kernsubstituenten im Nitro-p-phenylendiamin immer eine Abschwächung der Intensität der Färbungen verglichen mit der unsubstituierten Verbindung beobachtet.

Die Farbechtheit der Färbungen unter Säure- oder Lichteinwirkung ist bei den neuen erfindungsgemäßen Verbindungen gegenüber Nitro-p-phenylendiamin deutlich verbessert.

Die hier beschriebenen Verbindungen der allgemeinen Formel I lassen sich erfindungsgemäß nach einem allgemein anwendbaren Verfahren durch die Umsetzung eines 5-Chlor-2-nitro- oder 2-Chlor-5-nitro-p-phenylendiaminderivates mit einem Alkoholat in einem geeigneten Lösungsmittel darstellen. Als Lösungsmittel kann zweckmäßigerweise der Alkohol dienen, aus dem das entsprechende Alkoholat durch Zugabe von Natriummetall, Natriumhydroxid, Kaliumhydroxid, Calciumoxid oder Calciumhydroxid in situ erzeugt wird.

Die Reaktionstemperatur beträgt 80 bis 120°C, vorzugsweise 90 bis 100°C, bei einer Reaktionsdauer von 3 bis 24 Stunden.

Das gewünschte Produkt kann entweder durch Abkühlen der Lösung auskristallisiert werden, oder es wird durch Zugabe von Wasser ausgefällt. Ferner kann auch nach der Zugabe von Wasser und nach dem Entfernen von Nebenprodukten durch Extraktion mit Diethylether sodann mit Essigsäureethylester extrahiert werden, wobei die Verbindung der Formel I nach dem Einengen der Essigsäureethylesterphase in Form roter Kristalle oder als viskoses rotes Öl anfällt.

So wird beispielsweise die erfindungsgemäße Verbindung 2-Nitro-4-[(2'-hydroxyethyl)amino]-5-methoxyanilin durch die Reaktion von 2-Nitro-4-[(2'-hydroxyethyl)amino]-5-chlor-anilin mit Methanol, in dem zuvor eine geeignete Menge Natriummetall, Natriumhydroxid oder Kaliumhydroxid aufgelöst worden ist, hergestellt. In analoger Weise können hergestellt werden:

2-Nitro-4-[(2'-hydroxyethyl)-amino]-5-(2''-hydroxyethoxy)-anilin durch Reaktion von 2-Nitro-4-[(2'-hydroxyethyl)amino]-5-chlor-anilin mit Ethylenglykol/Natriumhydroxid oder Calciumoxid,

5-Nitro-4-[(2'-hydroxyethyl)amino]-2-methoxy-anilin durch Reaktion von 5-Nitro-4-[(2'-hydroxyethyl)amino]-2-chloranilin mit Methanol/Natriummetall,

2-Nitro-4-[(2',3'-dihydroxypropyl)amino]-5-methoxy-anilin durch Reaktion von 2-Nitro-4-[(2',3'-dihydroxypropyl)amino]-5-chlor-anilin mit Methanol/Natriummetall, und

2-Nitro-4-[(2'-hydroxyethyl)amino]-5-(2'',3''-dihydroxypropoxy)-anilin durch Reaktion von 2-Nitro-4-[(2'-hydroxyethyl)amino]-5-chlor-anilin mit Glyzerin/Natriumhydroxid.

Die angeführten Beispiele zeigen, daß die Reaktion allgemein zur Herstellung der erfindungsgemäßen Verbindungen nach Formel I anwendbar ist.

Die so dargestellten Verbindungen der allgemeinen Formel I können das Nitro-p-phenylendiamin in Haarfärbemitteln vorteilhaft ersetzen.

Gegenstand der vorliegenden Anmeldung sind daher ferner Mittel zur Färbung von Haaren mit einem Farbstoffgehalt und für Haarfärbemittel üblichen kosmetischen Zusätzen, gekennzeichnet durch den Gehalt an einer Verbindung der allgemeinen Formel I.

In den Haarfärbemitteln sollen die Verbindungen der allgemeinen Formel I, von denen die Verbindungen

2-Nitro-4-[(2'-hydroxyethyl)amino]-5-methoxy-anilin, 2-Nitro-4-[(2'-hydroxyethyl)amino]-5-(2''-hydroxy-ethoxy)-anilin und 5-Nitro-4-[(2'-hydroxyethyl)amino]-2-methoxy-anilin bevorzugt sind, in einer Konzentration von 0,01 bis 4,0 Gew.%, vorzugsweise 0,01 bis 1,0 Gew.%, enthalten sein.

Die erfindungsgemäßen Mittel zur Färbung von Haaren betreffen sowohl solche, die ohne Zugabe eines Oxidationsmittels angewendet werden, als auch solche, bei denen die Zugabe eines Oxidationsmittels erforderlich ist.

Bei den ersteren Haarfärbemitteln ohne Oxidationsmittelzugabe handelt es sich um diejenigen, die neben den Farbstoffen der angegebenen Formel I noch andere direkt auf das Haar aufziehende Farbstoffe enthalten können. Von diesen für die Haarfärbung bekannten Farbstoffen seien beispielsweise die folgenden erwähnt: Aromatische Nitrofarbstoffe, wie zum Beispiel 1,2-Diamino-4-nitro-benzol, 2-Amino-4-nitro-phenol, 1-[(2'-Hydroxyethyl)amino]-2-amino-4-nitro-benzol, 2-Nitro-4-[(2'-hydroxyethyl)amino]-anilin, 1-Methylamino-2-nitro-4-di-[(2'-hydroxy-ethyl)amino]-benzol, 1-[(2',3'-Dihydroxypropyl)-amino]-2-nitro-4-[ethyl-(2''-hydroxyethyl)amino]benzol,1-[(2',3'-Dihydroxypropyl)amino]-2-nitro-4-dimethylamino-benzol, N-[3-Nitro-4-(2',3'-dihydroxypropyl)aminophenyl]-pyrrolidin, 1-[(3'-Hydroxypropyl) amino]-2-nitro-4-[di-(2''-hydroxyethyl)amino]-benzol und 2,5-Di-[(2'-hydroxyethyl)-amino]-nitrobenzol; Triphenylmethanfarbstoffe, wie zum Beispiel Basic Violet 1 (C.I. 42 535), Azofarbstoffe, wie zum Beispiel Acid Brown 4 (C.I. 14 805); Anthrachinonfarbstoffe, wie zum Beispiel Disperse Violet 4 (C.I. 61 105), 1,4,5,8-Tetraaminoanthrachinon und 1,4-Diamino-anthrachinon, wobei die Farbstoffe dieser Klassen je nach Art ihrer Substituenten sauren, nichtionogenen oder basischen Charakter haben können.

Weitere geeignete direkt auf das Haar aufziehende Farbstoffe sind beispielsweise in dem Buch von J.C. Johnson, "Hair Dyes" Noyes Data Corp. Park-Ridge (USA) (1973) beschrieben. Diese bekannten Farbstoffe können in den erfindungsgemäßen Mitteln in einer Menge von etwa 0,01 bis 2,0 Gew.% enthalten sein.

Die Zubereitungsform der hier beschriebenen Haarfärbemittel auf der Basis von direkt auf das Haar aufziehenden Farbstoffen kann beispielsweise eine Lösung, inbesondere eine wäßrige Lösung oder wäßrigalkoholische Lösung, sein. Bevorzugte Zubereitungsformen sind weiterhin eine Creme, ein Gel oder eine Emulsion, wobei sie auch im Gemisch mit einem Treibgas oder mittels eine Pumpe versprüht werden können.

Die Farbstoffe der angegebenen Formel I sollen in diesen Färbemitteln ohne Oxidationsmittel-Zugabe in einer Konzentration von etwa 0,01 bis 2,0 Gew.%, vorzugsweise von 0,01 bis 1,0 Gew.%, enthalten sein. Der Gesamtgehalt an Farbstoffen liegt in den Grenzen von etwa 0,01 bis 3,0 Gew.%.

Der pH-Wert dieser Färbemittel liegt im Bereich von 3 bis 12, insbesondere bei pH 8 bis 11,5, wobei die Einstellung des gewünschten alkalischen pH-Wertes hauptsächlich mit Ammoniak erfolgt, jedoch auch mit organischen Aminen wie beispielsweise Monoethanol- oder Triethanolamin vorgenommen werden kann.

Ihre Anwendung erfolgt in üblicher Weise durch Aufbringen einer für die Haarfärbung ausreichenden Menge des Mittels auf die Haare, mit denen es eine Zeitlang, etwa 5 bis 30 Minuten, in Berührung bleibt. Anschließend wird mit Wasser, gegebenenfalls noch mit der wäßrigen Lösung einer schwachen organischen

Säure, gespült und getrocknet. Als schwache organische Säure können beispielsweise Essigsäure, Zitronensäure, Weinsäure und ähnliche Verwendung finden.

Die vorstehend beschriebenen Haarfärbemittel ohne Oxidationsmittel-Zugabe können selbstverständlich auch kosmetische Polymerisate enthalten, wodurch gleichzeitig mit der Färbung eine Festigung der Haare erreicht wird. Solche Mittel werden im allgemeinen als Tönungsfestiger oder Farbfestiger bezeichnet.

Von den für diesen Zweck in der Kosmetik bekannten Polymerisaten seien beispielsweise Polyvinylpyrrolidon, Polyvinylacetat, Polyvinylalkohol oder Polyacrylverbindungen wie Acrylsäure- beziehungsweise Methacrylsäurepolymerisate, basische Polymerisate von Estern aus diesen beiden Säuren und Aminoalkoholen beziehungsweise deren Salze oder Quaternisierungsprodukte, Polyacrylnitril, Polyvinyllactame sowie Copolymerisate aus derartigen Verbindungen wie Polyvinylpyrrolidon-Vinylacetat und dergleichen erwähnt.

Auch natürliche Polymere, wie Chitosan (entacetyliertes Chitin) oder Chitosanderivate, können für den genannten Zweck eingesetzt werden.

Die Polymerisate sind in diesen Mitteln in der üblichen Menge von 1 bis 5 Gew.% enthalten. Die pH-Werte der Mittel liegen im Bereich von etwa 6,0 bis 9,0.

Die Anwendung dieser Haarfärbemittel mit zusätzlicher Festigung erfolgt in bekannter und üblicher Weise durch Befeuchten des Haares mit dem Festiger, Festlegen (Einlegen) des Haares zur Frisur und anschließende Trocknung.

Selbstverständlich können die vorstehend beschriebenen Haarfärbemittel ohne Oxidationsmittel-Zugabe gegebenenfalls weitere gebräuchliche kosmetische Zusätze wie zum Beispiel Pflegestoffe, Netzmittel, Verdicker, Weichmacher und Parfümöle sowie auch andere, nachstehend für Oxidationshaarfärbemittel aufgeführte übliche Zusätze enthalten.

Zum Gegenstand der vorliegenden Erfindung gehören auch, wie eingangs erwähnt, diejenigen Haarfärbemittel, bei denen die Zugabe eines Oxidationsmittels erforderlich ist. Sie enthalten außer den Farbstoffen gemäß der angegebenen Formel I und gegebenenfalls bekannten direkt auf das Haar aufziehenden Farbstoffen noch zusätzlich bekannte Oxidationsfarbstoffe, die einer oxidativen Entwicklung bedürfen.

Bei diesen Oxidationsfarbstoffen handelt es sich hauptsächlich um aromatische p-Diamine und p-Aminophenole wie beispielsweise p-Toluylendiamin, p-Phenylendiamin, p-Aminophenol und ähnliche Verbindungen, welche zum Zwecke der Nuancierung der Färbungen mit sogenannten Modifieren, wie zum Beispiel m-Phenylendiamin, Resorcin, m-Aminophenol und anderen kombiniert werden.

Derartige zur Haarfärbung bekannte und übliche Oxidationsfarbstoffe sind unter anderem in dem Buch von E. Sagarin, "Cosmetics", Science and Technology (1957), Interscience Publishers Inc., New York, Seiten 503 ff. sowie in dem Buch von H. Janistyn, "Handbuch der Kosmetika und Riechstoffe" (1973) Seiten 338 ff., beschrieben. Die Oxidationshaarfarbstoffe können in einer Menge von 0,01 bis 2,0 Gew.% enthalten sein.

Mit Mischungen aus diesen Oxidationsfarbstoffen und den Farbstoffen gemäß der angegebenen Formel I lassen sich neben reinen Modetönen auch modische Blond- und Brauntöne erhalten.

Die Farbstoffe gemäß der Formel I sind in diesen Färbemitteln mit Oxidationsmittel-Zugabe in einer Konzentration von etwa 0,01 bis 4,0 Gew.%, vorzugsweise 0,02 bis 2,0 Gew.%, enthalten. Der Gesamtgehalt an Farbstoffen in diesen Färbemitteln beträgt etwa 0,1 bis 5,0 Gew.%.

Oxidationshaarfärbemittel sind im allgemeinen alkalisch eingestellt, vorzugsweise auf pH-Werte von etwa 8,0 bis 11,5, wobei die Einstellung insbesondere mit Ammoniak erfolgt. Es können dazu aber auch organische Amine, zum Beispiel Monoethanolamin oder Triethanolamin, Verwendung finden. Als Oxidationsmittel zur Entwicklung der Haarfärbungen kommen hauptsächlich Hydrogenperoxyd und dessen Additionsverbindungen in Betracht. Die Zubereitungsform dieser Haarfärbemittel kann die gleiche wie bei Haarfärbemitteln ohne Oxidationsmittelzugabe sein. Vorzugsweise ist sie die einer Creme oder eines Gels.

Übliche Zusätze in Cremes, Emulsionen oder Gelen sind zum Beispiel Lösungsmittel wie Wasser, niedere aliphatische Alkohole, beispielsweise Ethanol, Propanol, Isopropanol, Glycerin oder Glykole wie Ethylenglykol und Propylenglykol, oder auch Glykolether, weiterhin Netzmittel oder Emulgatoren aus den Klassen der anionischen, kationischen, amphoteren oder nichtionogenen oberflächenaktiven Substanzen wie Fettalkoholsulfate, Fettalkoholethersulfate, Alkylsulfonate, Alkylbenzolsulfate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Bentonit, Stärke, Polyacrylsäure, Cellulosederivate wie Carboxymethylcellulose, Alginate, Vaseline, Paraffinöl und Fettsäuren sowie außerdem Pflegestoffe wie Lanolinderivate, Cholesterin, Pantothensäure und Betain, weiterhin Parfümöle und Komplexbildner. Die erwähnten Bestandteile werden in den für solche Zwecke üblichen Mengen verwendet, zum Beispiel die Netzmittel und Emulgatoren in Konzentrationen von etwa 0,5 bis 30 Gew.%, während die Verdicker in einer Menge von etwa 0,1 bis 25 Gew.% in den Zubereitungen enthalten sein können.

Die Anwendung der genannten Zubereitungen, bei denen die Zugabe eines Oxidationsmittels erforderlich ist, erfolgt in bekannter Weise, indem man die Haarfärbemittel vor der Behandlung mit dem Oxidationsmittel vermischt und eine zur Färbung des Haares ausreichende Menge der Mischung, im allgemeinen etwa 50 bis 150 ml, auf das Haar aufträgt.

Nach einer für die Haarfärbung ausreichenden Einwirkungszeit, welche üblicherweise etwa 10 bis 45 Minuten beträgt, wird mit Wasser, gegebenenfalls anschließend mit der wäßrigen Lösung einer schwachen organischen Säure, wie zum Beispiel Zitronensäure oder Weinsäure, gespült und getrocknet.

Hinsichtlich der färberischen Möglichkeiten bieten die erfindungsgemäßen Haarfärbemittel je nach Art und Zusammensetzung der Farbkomponenten eine breite Palette verschiedener Farbnuancen, die sich von

natürlichen Farbtönen bis hin zu hochmodischen, leuchtenden Nuancen erstreckt. Hierbei werden die Färbemittel je nach Zusammensetzung entweder in Verbindung mit Hydrogenperoxid oder auch ohne Oxidationsmittel angewandt.

Die nachstehenden Beispiele sollen den Anmeldungsgegenstand erläutern, ohne ihn darauf zu beschränken.

## HERSTELLUNGSBEISPIELE

### Beispiel 1

Herstellung von 2-Nitro-4-[(2'-hydroxyethyl)amino]-5-methoxy-anilin

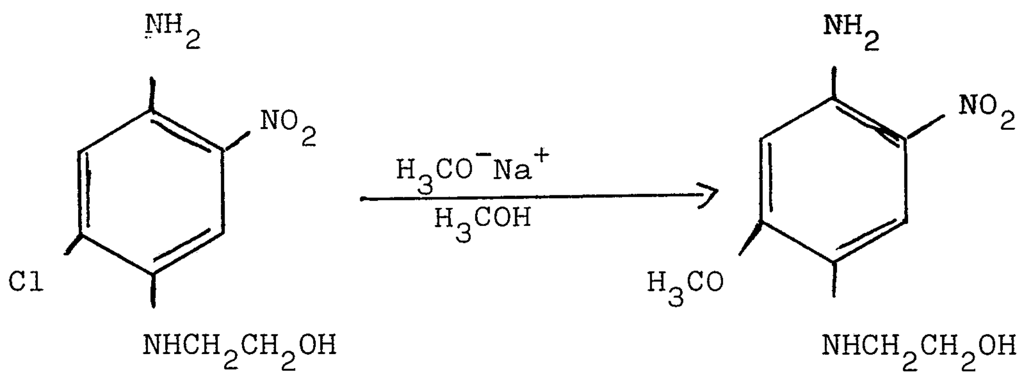


4,0g (0,17 mol) Natrium werden in 250 ml Methanol aufgelöst. Danach werden 11,6g (50 mmol) 2-Nitro-4-[(2'-hydroxyethyl)amino]-5-chlor-anilin zugesetzt. Die Mischung wird 24 Stunden lang unter Rückfluß gekocht. Nach dem Abkühlen wird das Reaktionsprodukt mit Wasser ausgefällt, sodann abgesaugt und mit Wasser gewaschen. Nach dem Trocknen über Calciumchlorid erhält man 8,4g (37 mmol; 74 % der Theorie) eines rotbraunen Produkts, das nach dem Umkristallisieren aus Essigsäureethylester bei 173-175°C schmilzt.

CHN - Analyse:

| | | %C | %H | %N |
|---|---|---|---|---|
| $C_9H_{13}N_3O_4$ | berechnet: | 47,57 | 5,77 | 18,49 |
| | gefunden: | 47,08 | 5,80 | 18,29 |

Beispiel 2

Herstellung von 2-Nitro-4-[(2'-hydroxyethyl)amino]-5-(2''-hydroxyethoxy)-anilin

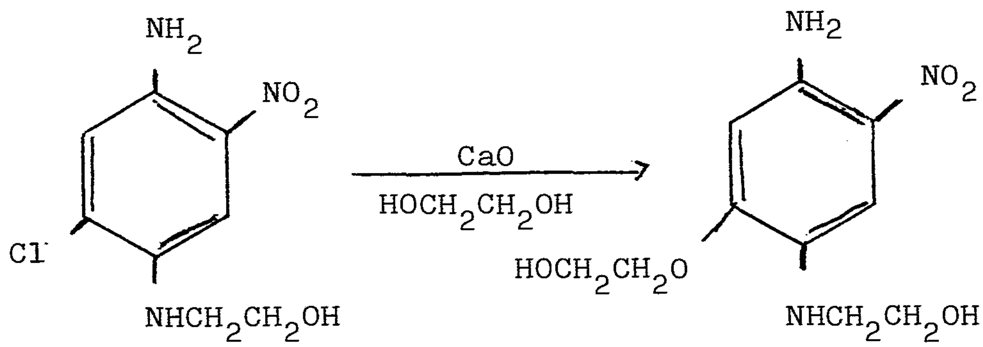


4,8g (86 mmol) Calciumoxid werden mit 52 ml Ethylenglykol und 10 g 2-Nitro-4-[(2'-hydroxyethyl)amino]-5-chloranilin drei Stunden lang auf 120°C erwärmt. Nach dem Abkühlen wird die Mischung mit 100 ml gesättigter Kochsalzlösung verdünnt und mit 4 x 200 ml Diethylether extrahiert. Darauf wird die wäßrige Phase mit 8 x 100 ml Essigsäureethylester extrahiert. Nach dem Trocknen über Magnesiumsulfat wird die Essigsäureethylesterphase auf ein kleines Volumen eingeengt. Das gewünschte Produkt fällt als roter Niederschlag aus. Man erhält 2,0 g (40 % der Theorie) rote Kristalle vom Schmelzpunkt 154-155°C.

CHN - Analyse

| | | %C | %H | %N |
|---|---|---|---|---|
| $C_{10}H_{15}N_3O_5$ | berechnet: | 46,69 | 5,88 | 16,33 |
| | gefunden: | 46,67 | 5,83 | 15,93 |

$^1$H-NMR-Spektrum (Dimethylsulfoxid, $\delta$ in ppm):

3,10 (q), 3,65 (q), 3,80 (q), 4,05 (q), 4,85 (tr), 4,95 (tr), 5,05 (tr), 6,35 (s), 6,90 (s), 7,30 (s)
Standard: Tetramethylsilan
Multiplizitäten: s = Singulett, tr = Triplett,
q = Quartett

Beispiel 3

Herstellung von 5-Nitro-4-[(2'-hydroxyethyl)amino]-2-methoxy-anilin

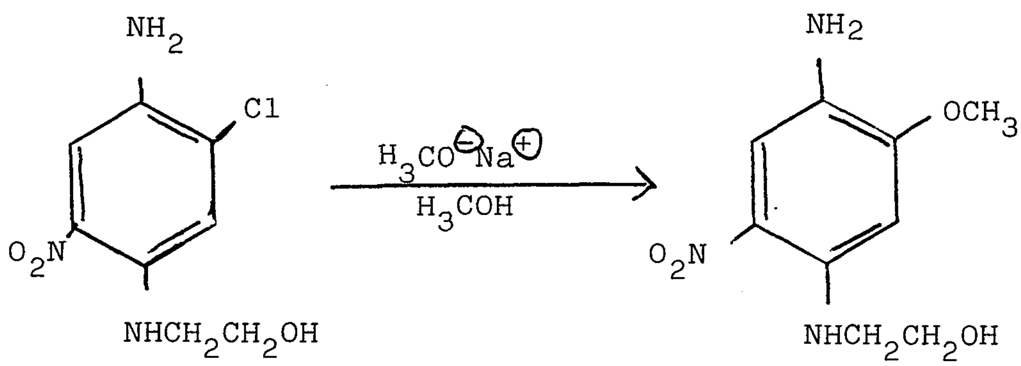


0,11g (4,7 mmol) Natrium werden in 10 ml Methanol aufgelöst. Nach der Zugabe von 0,24g (1,0 mmol) 5-Nitro-4-[(2'-hydroxyethyl)amino]-2-chlor-anilin wird der Ansatz 24 Stunden lang unter Rückfluß gekocht. Das beim Abkühlen auskristallisierende Produkt wird abgesaugt, mit Methanol gewaschen und über Calciumchlorid getrocknet. Man erhält 0,20g (0,9 mmol; 85% der Theorie) orangebraunes Produkt vom Schmelzpunkt 157-158°C.

CHN-Analyse

| | | %C | %H | %N |
|---|---|---|---|---|
| $C_9H_{13}N_3O_4$ | berechnet: | 47,57 | 5,77 | 18,49 |
| | gefunden: | 47,79 | 5,63 | 18,05 |

Beispiel 4

Herstellung von 2-Nitro-4-[(2',3'-dihydroxypropyl)amino]-5-methoxy-anilin

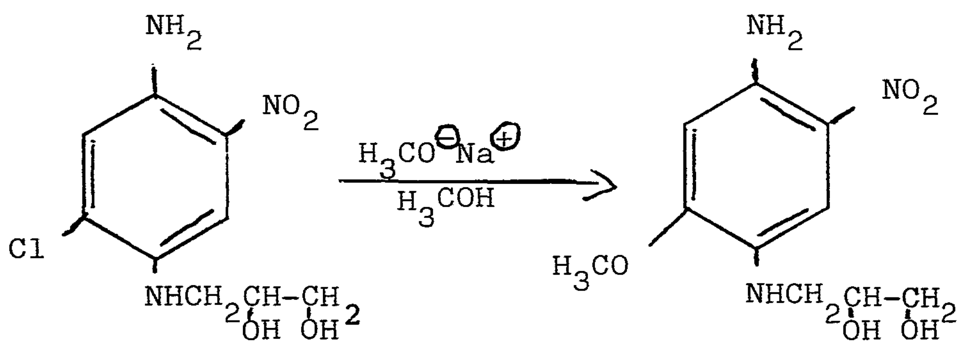


0,15g (6,5 mmol) Natrium werden in 10 ml Methanol aufgelöst. Zu dieser Lösung werden 0,5g (1,9 mmol) 2-Nitro-4-[(2',3'-dihydroxypropyl)amino]-5-chlor--anilin gegeben. Der Ansatz wird 20 Stunden lang auf 80°C erwärmt. Danach werden 20 ml Wasser zugesetzt. Das Produkt wird mit Essigsäureethylester extrahiert. Nach dem Trocknen über Magnesiumsulfat erhält man daraus 0,3g (1,2 mmol; 60 % der Theorie) eines roten, halbfesten Öls.

Dieses Produkt vermag das Haar in einem ähnlich roten Farbton zu färben, wie das Produkt nach Beispiel 1.

Beispiel 5

Herstellung von 2-Nitro-4-[(2'-hydroxyethyl)amino]-5-(2",3"-dihydroxypropoxy)-anilin

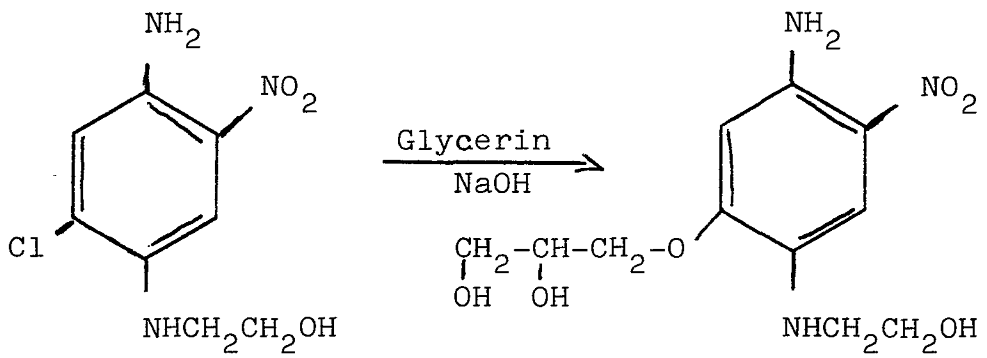


2,4g (60 mmol) Natriumhydroxid werden in 50 ml Glyzerin aufgelöst. Dann werden 4,6g (29 mmol) 2-Nitro-4-[(2'-hydroxyethyl)-amino]-5-chlor-anilin zugegeben. Die Mischung wird 6 Stunden lang auf 100°C erwärmt und nach dem Abkühlen mit 200 ml Wasser verdünnt. Zur Entfernung eines Nebenprodukts wird die Mischung mehrmals mit Diethylether extrahiert. Das gewünschte Reaktionsprodukt läßt sich durch Extraktion mit Essigsäureethylester isolieren. Nach dem Trocknen über Magnesiumsulfat und Filtration erhält man daraus 2,2g (38 % der Theorie) rotes Öl.

Das Produkt färbt das Haar in einem reinen, roten Farbton.

## BEISPIELE FÜR HAARFÄRBEMITTEL

### Beispiel 6

Färbelösung

| | |
|---|---|
| 0,3 g | 2-Nitro-4[(2'-hydroxyethyl)amino]-5-methoxy-anilin |
| 2,0 g | Laurylalkoholdiglykolethersulfat-Natriumsalz, 28%-ige wäßrige Lösung |
| 2,0 g | Ammoniak, 25%-ige wäßrige Lösung |
| 95,7 g | Wasser |
| 100,0 g | |

Gebleichtes menschliches Naturhaar wird 20 Minuten lang bei Raumtemperatur mit der Lösung nach Beispiel 6 behandelt. Danach wird mit Wasser ausgespült und getrocknet. Das Haar ist leuchtend rot gefärbt.

### Beispiel 7

Farbfestiger

| | |
|---|---|
| 0,1 g | 2-Nitro-4-[(2'-hydroxyethyl)amino]-5-(2''-hydroxyethoxy)-anilin |
| 2,0 g | Polyvinylpyrrolidon |
| 0,1 g | Glyzerin |
| 40,0 g | Isopropanol |
| 57,8 g | Wasser |
| 100,0 g | |

Weiße menschliche Haare werden mit der Farbfestiger-Lösung zur Frisur eingelegt und getrocknet. Das Haar ist rot gefärbt und gefestigt.

Beispiel 8

Oxidationshaarfärbemittel in Cremeform

| | |
|---|---|
| 0,50 g | 5-Nitro-4-[(2'-hydroxyethyl)amino]-2--methoxy-anilin |
| 0,40 g | 2,5-Diaminotoluolsulfat |
| 0,26 g | Resorcin |
| 0,40 g | 4-Aminophenol |
| 0,13 g | 2,4-Diaminoanisol |
| 0,30 g | Natriumsulfit, wasserfrei |
| 0,30 g | Ethylendiamintetraessigsäure-dinatriumsalz |
| 15,00 g | Cetylalkohol |
| 3,50 g | Laurylalkohol-diglykolethersulfat-Natrium-salz, 28%-ige wäßrige Lösung |
| 6,00 g | Ammoniak, 25%-ige wäßrige Lösung |
| 73,21 g | Wasser |
| 100,00 g | |

50 ml des vorstehenden Haarfärbemittels werden kurz vor der Anwendung mit 50 ml wäßriger Hydrogenperoxidlösung (6%-ig) gemischt. Das Gemisch wird anschließend auf graue menschliche Haare aufgetragen und 30 Minuten bei einer Temperatur von 40°C einwirken gelassen. Nach dem Spülen der Haare mit Wasser und anschließendem Trocknen haben diese einen Palisanderton angenommen.

Alle vorstehend angegebenen Prozentangaben stellen Gewichtsprozente dar.

Patentansprüche

1. Nitro-p-phenylendiaminderivat der allgemeinen Formel I

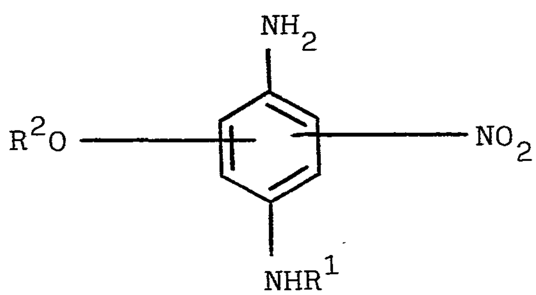

(I),

worin die Gruppe $R^2O$ sich in p-Stellung zur Nitrogruppe befindet und der Rest $R^1$

- einen Alkylrest mit 1 bis 6 Kohlenstoffatomen oder

- einen Mono- oder Polyhydroxyalkylrest mit 1 bis 6 Kohlenstoffatomen und bis zu 3 Hydroxylgruppen oder

- einen Aminoalkylrest oder einen Mono- oder Dialkylaminoalkylrest der allgemeinen Formel $C_mH_{2m}N(C_nH_{2n})_pH_{(2-p)}$, wobei m und n unabhängig voneinander eine ganze Zahl von 1 bis 6 darstellen und p eine ganze Zahl von 0 bis 2 ist,

bedeutet und der Rest $R^2$ unabhängig vom Rest $R^1$

- einen Alkylrest mit 1 bis 6 Kohlenstoffatomen oder

- einen Mono- oder Dihydroxyalkylrest mit 1 bis 6 Kohlenstoffatomen

darstellt.

2. 2-Nitro-4-[(2'-hydroxyethyl)amino]-5-methoxy-anilin.

3. 2-Nitro-4-[(2'-hydroxyethyl)amino]-5-(2''-hydroxy-ethoxy)-anilin.

4. 5-Nitro-4-[(2'-hydroxyethyl)amino]-2-methoxy-anilin.

5. 2-Nitro-4-[(2',3'-dihydroxypropyl)amino]-5-methoxy-anilin.

6. 2-Nitro-4-[(2'-hydroxyethyl)amino]-5-(2'',3''-dihy-droxypropoxy)-anilin.

7. Verfahren zur Herstellung der Verbindungen nach den Ansprüchen 1 bis 6, dadurch gekennzeichnet, daß man ein 5-Chlor-2-nitro- oder 2-Chlor-5-nitro-p-phenylendiaminderivat mit einem Alkoholat in einem geeigneten Lösungsmittel umsetzt.

8. Verfahren nach Anspruch 7, dadurch gekennzeichnet, daß man das Alkoholat durch Auflösen von Natrium-metall, Natriumhydroxid, Kaliumhydroxid, Calcium-oxid oder Calciumhydroxid in dem entsprechenden Alkohol in situ erzeugt.

9. Verfahren nach den Ansprüchen 7 und 8, dadurch gekennzeichnet, daß die Reaktionstemperatur 80-120°C beträgt.

10. Verfahren nach den Ansprüchen 7 bis 9, dadurch gekennzeichnet, daß die Reaktionsdauer 3 bis 24 Stunden beträgt.

0203446

11. Verfahren nach den Ansprüchen 7 bis 10, dadurch gekennzeichnet, daß man das gewünschte Produkt durch Abkühlen auskristallisiert oder durch Zugabe von Wasser ausfällt oder nach der Zugabe von Wasser zunächst durch Extraktion mit Diethylether die Nebenprodukte entfernt und aus dem Rückstand anschließend mit Essigsäureethylester die Verbindung der Formel I extrahiert.

12. Mittel zur Färbung von Haaren mit einem Farbstoffgehalt und für Haarfärbemittel üblichen kosmetischen Zusätzen, dadurch gekennzeichnet, daß es eine Verbindung nach den Ansprüchen 1 bis 6 enthält.

13. Mittel nach Anspruch 12, dadurch gekennzeichnet, daß der Farbstoff nach den Ansprüchen 1 bis 6 in einer Menge von 0,01 bis 4,0 Gew.% enthalten ist.

14. Mittel nach den Ansprüchen 12 und 13, dadurch gekennzeichnet, daß es mindestens einen der bekannten direkt auf das Haar aufziehenden Farbstoffe enthält.

15. Mittel nach Anspruch 14, dadurch gekennzeichnet, daß der direkt auf das Haar aufziehende Farbstoff ausgewählt ist aus 2-Amino-4-nitro-phenol, 1-[(2'-Hydroxyethyl)amino]-2-amino-4-nitro-benzol, 2-Nitro-4-[(2'-hydroxyethyl)amino]-anilin, 1-Methylamino-2-nitro-4-di-[(2'-hydroxyethyl)-amino]-benzol, 2,5-Di-[(2'-hydroxyethyl)amino]-nitro-benzol, Acid Brown 4 (C.I. 14 805), Disperse Violet 4 (C.I. 61 105) oder Basic Violet 1 (C.I. 42 535).

16. Mittel nach den Ansprüchen 12 bis 15, dadurch gekennzeichnet, daß es in Form eines Haarfärbemittels mit zusätzlicher Haarfestigung vorliegt und mindestens ein in der Kosmetik üblicherweise verwendetes Polymerisat enthält.

17. Mittel nach Anspruch 16, dadurch gekennzeichnet, daß das Polymerisat ausgewählt ist aus Polyvinylpyrrolidon, Polyvinylacetat, Polyvinylalkohol, Polyacrylsäure, Polymethacrylsäure, Polyacrylnitril, Polyvinyllactam oder Copolymerisaten aus diesen Verbindungen.

18. Mittel nach den Ansprüchen 12 bis 15, dadurch gekennzeichnet, daß es mindestens einen der bekannten Oxidationshaarfarbstoffe enthält.

19. Mittel nach Anspruch 18, dadurch gekennzeichnet, daß der Oxidationshaarfarbstoff in einer Konzentration von 0,01 bis 2,0 Gew.% enthalten ist und ausgewählt ist aus p-Toluylendiamin, p-Phenylendiamin, p-Aminophenol, m-Phenylendiamin, Resorcin und m-Aminophenol.

20. Mittel nach den Ansprüchen 18 und 19, dadurch gekennzeichnet, daß es einen pH-Wert von 8,0 bis 11,5 aufweist.

Europäisches Patentamt

**EUROPÄISCHER RECHERCHENBERICHT**

0203446

Nummer der Anmeldung

EP 86106488.9

**EINSCHLÄGIGE DOKUMENTE**

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| | DE - B2 - 1 924 249 (BRISTOL-MYERS) | | C 07 C 93/14 |
| D,X | * Anspruch 1 * | 1 | C 09 B 51/00 |
| A | * Spalte 4, Zeile 28 - Spalte 5, Zeile 37; Beispiel 11 * | 12-14, 16-18, 20 | A 61 K 7/13 |
| | -- | | |
| | GB - A - 1 093 506 (REVLON INC.) | | |
| X | * Seite 2, Zeilen 1-13 * | 1 | |
| A | * Ansprüche 16,17 * | 12 | |
| | -- | | |
| A | DE - A1 - 3 141 019 (L'OREAL) * Ansprüche 1-3,5,6,8,9,11,12 * | 1,12-14,16-20 | |
| | -- | | |
| A | GB - A - 2 108 994 (L'OREAL) * Zusammenfassung; Seite 3, Zeile 14 - Seite 4, Zeile 45; Seite 18, Zeilen 1-6 * | 1,7, 12-14, 16-20 | **RECHERCHIERTE SACHGEBIETE (Int. Cl.4)** |
| | -- | | C 07 C 93/00 |
| | | | C 09 B |
| A | GB - A - 2 139 218 (BRISTOL-MYERS) * Ansprüche 9-17; Beispiel 1 * | 1,8-11,18 | A 61 K |
| | ---- | | |

Der vorliegende Recherchenbericht wurde für alle Patentansprüche erstellt.

| Recherchenort | Abschlußdatum der Recherche | Prüfer |
|---|---|---|
| WIEN | 09-09-1986 | KÖRBER |

**KATEGORIE DER GENANNTEN DOKUMENTEN**
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82